# EUROPEAN PATENT SPECIFICATION

(11) **EP 2 293 677 B1**
(45) Date of publication and mention of the grant of the patent: **15.11.2017**
(21) Application number: 09762711.1
(22) Date of filing: 12.06.2009
(51) Int. Cl.: A23L 33/00, A23C 9/12, A23C 9/123, A23C 9/13, A23C 9/20, A23L 33/10, A23L 33/21, A61K 35/74, A61P 1/12, A61P 11/06, A61P 17/00, A61K 31/702, A61K 31/733, A61K 35/744, A61K 35/745, A23L 33/135

(54) **NUTRITIONAL COMPOSITION FOR INFANTS DELIVERED VIA CAESAREAN SECTION**
ERNÄHRUNGSZUSAMMENSETZUNG FÜR DURCH KAISERSCHNITT ENTBUNDENE SÄUGLINGE
COMPOSITION NUTRITIONNELLE DESTINÉE AUX NOURRISSONS NÉS PAR CÉSARIENNE

(30) Priority: 13.06.2008 WO PCT/NL2008/050375; 31.10.2008 EP 08168054
(43) Date of publication of application: 16.03.2011
(73) Proprietor: N.V. Nutricia, 2712 HM Zoetermeer (NL)
(72) Inventor: SCHMITT, Joachim, 63768 Hoesbach (DE); PERRIN, Emmanuel, F-59299 Boeschepe (FR); STAHL, Bernd, 61191 Rosbach-Rodheim (DE); BOEHM, Günther, 61209 Echzell (DE)
(74) Representative: Nederlandsch Octrooibureau
(86) International application number: PCT/NL2009/050332
(87) International publication number: WO 2009/151330

(56) References cited:
- EP-A- 1 364 586
- EP-A- 1 597 978
- EP-A- 1 776 877
- WO-A-2006/087391
- WO-A-2007/045502
- US-A1- 2004 072 794
- US-A1- 2006 018 890
- US-A1- 2006 233 773
- ANONYMOUS: "Life Start Dairy" INTERNET CITATION, February 2006 (2006-02), XP002369353
- HEYMAN M ET AL: "Effects of specific lactic acid bacteria on the intestinal permeability to macromolecules and the inflammatory condition" ACTA PAEDIATRICA, UNIVERSITETSFORLAGET, OSLO, NO, vol. 94, no. Suppl. 449, 1 October 2005 (2005-10-01), pages 34-36, XP002480544 ISSN: 0803-5253
- KIRJAVAINEN P V ET AL: "Probiotic bacteria in the management of atopic disease: Underscoring the importance of viability" JOURNAL OF PEDIATRIC GASTROENTEROLOGY AND NUTRITION, RAVEN PRESS, NEW YORK, NY, US, vol. 36, no. 2, 1 January 2003 (2003-01-01), pages 223-227, XP009100997 ISSN: 0277-2116
- MCVAY MARCENE R ET AL: "Formula fortified with live probiotic culture reduces pulmonary and gastrointestinal bacterial colonization and translocation in a newborn animal model" JOURNAL OF PEDIATRIC SURGERY JAN 2008, vol. 43, no. 1, January 2008 (2008-01), pages 25-29, XP002506653 ISSN: 1531-5037
- FANARO SILVIA ET AL: "Galacto-oligosaccharides and long-chain fructo-oligosaccharides as prebiotics in infant formulas: a review" ACTA PAEDIATRICA, vol. 94, no. S449, October 2005 (2005-10), pages 22-26, XP002506654 OSLO, NORWAY ISSN: 0803-5326
- GR\NLUND M M ET AL: "FECAL MICROFLORA IN HEALTHY INFANTS BORN BY DIFFERENT METHODS OF DELIVERY: PERMANENT CHANGES IN INTESTINAL FLORA AFTER CESAREAN DELIVERY" JOURNAL OF PEDIATRIC GASTROENTEROLOGY AND NUTRITION, RAVEN PRESS, NEW YORK, NY, US, vol. 28, no. 1, January 1999 (1999-01), pages 19-25, XP009084526 ISSN: 0277-2116

## Description

### FIELD OF THE INVENTION

The present invention relates to methods for feeding of infants delivered via caesarean section and to compositions to be administered to infants delivered via caesarean section.

### BACKGROUND OF THE INVENTION

Before birth the intestinal tract of the infant is normally sterile. During vaginal delivery the intestinal tract of the infant is inoculated with vaginal and/or faecal bacteria of the mother, resulting in a colonization of the infant's gastrointestinal tract by bacteria originating from the mother. A maternal healthy intestinal microbiota has numerous positive effects on the infant, such as a reduced incidence of infections and a strengthened immune system.

In infants delivered via caesarean section the colonization by intestinal bacteria is delayed and occurs by bacteria present in the hospital environment, resulting in the development of a different, less optimal intestinal microbiota. Grönlund et al, 1999, JPGN 28:19-25, disclose that there are differences in intestinal flora after caesarean delivery compared to vaginally born infants. The intestinal microbiota of caesarean delivered infants comprises less bacteria, less beneficial bacteria and less species of beneficial bacteria, compared to intestinal microbiota of infants born via the vaginal route. In particular, the profile and content of lactic acid producing bacteria such as *Bifidobacterium* species of the microbiota of infants delivered via caesarean section is different from the intestinal profile and content of *Bifidobacterium* species of infants delivered via the vaginal route. These differences in microbiota persist well into childhood.

Infant formulae are normally designed to mimic the development of an intestinal microbiota in an infant receiving human breast milk, with the implication that all infants react similar to human breast milk and infant formula. However, the sub-population of infants delivered via caesarean section will react differently because the colonization is delayed and less optimal.

EP 1776877 discloses the use of at least two different microorganisms, or at least one microorganism and at least one indigestible oligosaccharide or at least two different *Bifidobacterium* species, subspecies or strains for the manufacture of a composition for enteral administration to an infant delivered via caesarean section. These bacteria are living bacteria.

WO 2007/046698 discloses the use of a composition comprising non-digestible oligosaccharide for the manufacture of a composition for enteral administration to an infant delivered via caesarean section. Optionally lactic acid bacteria are present.

Natren® produces the probiotic product Life Start® which is designed specifically for infants and suitable for infants delivered via caesarean section. Life Start® comprises living *Bifidobacterium infantis.*

Heyman et al, 2005, Acta Paediatrica 94:34-36, disclose the use of non-live micro-organisms in fermented infant formula intended for healthy infants with a positive effect on intestinal function.

US 2006/018890 is directed to the use of treating respiratory infections and acute otitis media in infants by administering bifidobacteria and an adherence promoting strain. Living bacteria are preferred and the experimental data shown use living *Bifidobacterium lactis* and *Lactobacillus* GG.

EP 1364586 discloses the use of *Lactobacillus paracasei* and *Bifidobacterium lactis* to promote oral tolerance. Optionally the bacteria are dead. Optionally these bacteria are added to fermented products not including infant formula.

Kirjavainen et al, 2003, JPGN 36:223-227, disclose that using viable, but not heat inactivated LGG is a potential approach for the management of atopic eczema and cow's milk allergy. The tested products are not fermented compoitions.

EP 1597978 discloses a synergistic effect between polyfructose and galacto-oligosaccharide when fermented by infant's faeces.

US 2006/0233773 discloses the use of *Lactobacillus* GG for preventing or reducing the development of respiratory allergies. These bacteria are living.

Mcvay et al, 2008, J Pediatr Surg 43:25-29, disclose that a formula with live probiotic bacteria was superior compared with chemically acidified, *Lactococcis lactis* fermented infant formula and control infant formula in reducing pulmonary and gastrointestinal bacterial colonization. The formulae were tested in rabbit pups delivered via caesarean section.

### SUMMARY OF THE INVENTION

Animal experiments showed that already within two hours after birth the intestine of a vaginally born infant shows an immunological response to bacteria, whereas in caesarean section delivered infants no such fast immunological response is observed. This response is initiated by the immunogenic factors of the bacteria and is indicative for tolerance induction against these bacteria, thereby enabling a fast colonisation of the gut. It is believed that this fast immunological response is very important for a healthy development of the infant. Hence, it is particularly desirable to have similar effects in infants born via caesarean section, particularly the tolerance induction and fast colonisation of the gut by bacteria.

The inventors recognized that early exposure of the intestine of the newborn infant that was delivered by caesarean section, to a milk-derived product which has been fermented by lactic acid producing bacteria, induces an intestinal tolerance for these bacteria similar to that of vaginally born infants, enabling a fast colonisation of the intestine as in vaginally born infants.

However, the tolerance inducing effects of the fermented milk alone is insufficient to enable a sufficient intestinal colonisation. Hence, it is particularly important that the present composition comprises a mixture of oligosaccharides that efficiently improve the colonisation of the gut by lactic acid producing bacteria.

Because the intestinal microbiota plays a crucial role in the development of the infant, in particularly in the stimulation of the immune system, susceptibility for atopic diseases and resistance against infections, it is of utmost importance to stimulate a fast and healthy development of the intestinal microbiota of infants born via caesarean section.

Caesarean section delivered infants are delivered in a hospital environment, which is a risk for pathogenic infection and/or diarrhoea due to the occurrence of nosocomial bacteria. Additionally, the impaired development of a healthy intestinal microbiota results in faster colonisation of pathogenic bacteria compared to a situation where the infants intestinal tract is inoculated by maternal bacteria. The present invention particularly aims to provide a composition which decreases the incidence and severity of infections and/or diarrhoea in infants born via caesarean section, by inducing tolerance of the infant's gut for beneficial bacteria, by stimulating the growth of beneficial bacteria, preferably lactic acid producing bacteria, and/or by decreasing the growth of adverse bacteria. Hence the present composition can be advantageously used to treat and/or prevent infections in infants born via caesarean section.

Caesarean section delivered infants have an increased risk in atopic diseases such as food allergy, asthma, atopic dermatitis, and/or allergic rhinitis. The present invention particularly aims to provide a composition which decreases the incidence and severity of atopic diseases such as atopic eczema (or atopic dermatitis), allergy and/or asthma in infants born via caesarean section, by improving the intestinal colonization of beneficial bacteria. Hence the present invention can be advantageously used to treat and/or prevent allergy in infants born via caesarean section.

The milk-derived product which has been fermented by lactic acid producing bacteria comprises fragments of lactic acid producing bacteria or/and products excreted by lactic acid producing bacteria, such as glycoproteins, glycolipids, peptidoglycan, lipoteichoic acid (LTA), flagellae, lipoproteins, capsular polysaccharides and/or DNA. Without wishing to be bound by theory, it is believed by the present inventors that these immunogenic molecules induce the tolerance of the intestinal tract against colonisation with lactic acid producing bacteria. Induction of tolerance against lactic acid producing bacteria in the intestinal tract results in a faster colonisation by the desired bacteria, while on the other hand the absence of living cells in the product results in an increased safety and improved product technological properties. The safety advantage is especially important in case of caesarean section delivered infants, which are more vulnerable to infections.

The composition comprises at least two non-digestible oligosaccharides. The presence of non-digestible oligosaccharides stimulates the growth of lactic acid producing bacteria, such as lactobacilli and/or bifidobacteria, reduces the growth of non beneficial bacteria in the gastro-intestinal tract and/or directly advantageously stimulates the immune system. This results in a higher colonisation of the beneficial bacteria. Hence, the presence of both non-digestible oligosaccharides and the milk-derived product obtained by fermentation with lactic acid producing bacteria acts synergistically and advantageously results in both a faster and a higher colonization with lactic acid producing bacteria, such as lactobacilli and/or bifidobacteria.

The presence of at least two different non-digestible oligosaccharides results in a microbiota that is more diverse in respect of different lactic acid producing bacteria species as is the case in vaginally born infants. The presence of at least two different non-digestible oligosaccharides and the milk-derived product obtained by fermentation with lactic acid producing bacteria act synergistically and advantageously results in a faster, as well as a higher as well as a more diverse colonisation with lactic acid producing bacteria, especially with lactobacilli and/or bifidobacteria.
It was observed that a composition comprising at least two different non-digestible oligosaccharides and a milk-derived product obtained by fermentation with lactic acid producing bacteria had a synergistic effect on the immune response as tested in two animal models: a vaccination model and an allergy model.

### DETAILED DESCRIPTION OF THE PREFERRED EMBODIMENTS

The present invention relates to a method for providing nutrition to an infant delivered via caesarean section, and a composition and its uses, according to the appended claims. In the context of this invention the term 'milk substrate', also denoted as 'aqueous substrate' or simply 'substrate' is the material that is subjected to fermentation by lactic acid producing bacteria. In the context of this invention the term 'milk-derived product' is the product that results from the fermentation of substrate by lactic acid producing bacteria and is also denoted as 'fermented product'.
Fermentation is the process of deriving energy from the oxidation of carbohydrates, such as the lactose present in milk, using an endogenous electron acceptor, which is usually an organic compound. This is in contrast to cellular respiration, where electrons are donated to an exogenous electron acceptor, such as oxygen, via an electron transport chain.
In the present invention fermentation of a milk-derived product by lactic acid producing bacteria has the common meaning of the conversion of carbohydrates present in the milk-derived product to organic acids. These organic acids formed may comprise, besides lactic acid, also other organic acids such as acetate. The carbohydrate that is fermented is preferably lactose.

Wherever herein below reference is made to the nutritional composition of the present invention or further or preferred embodiments of the nutritional composition of the present invention are specified, this is also applicable to the use according to the present invention.

### Caesarean section

The present invention relates to the enteral administration of a composition comprising a milk-derived product obtainable by fermentation of a milk substrate by lactic acid producing bacteria to infants delivered via caesarean section. A caesarean section (c-section) is a surgical procedure where an infant is delivered through an incision made in the mother's abdominal wall, and then through the wall of the uterus. A caesarean section is usually performed when it is safer for the mother or the infant than a vaginal delivery. Alternatively, a woman may choose to have a caesarean section rather than deliver her infant vaginally.

### Fermented milk-derived product by lactic acid producing bacteria.

A nutritional composition of the present invention or for use according to the present invention comprises a milk-derived product, which is a milk substrate that is fermented by lactic acid producing bacteria, and said milk substrate comprising at least one selected from the group consisting of milk, whey, whey protein, whey protein hydrolysate, casein, casein hydrolysate and/or lactose. The composition of the present invention preferably comprises a milk-derived product which is obtainable by a process comprising the following steps:
a. inoculating lactic acid producing bacteria in an aqueous medium comprising milk substrate in amount of between 1x10² to 5x10¹⁰ cfu/ml, said aqueous medium having a pH of approximately between 4 and 8, and comprising at least one milk substrate selected from the group consisting of milk, whey, whey protein, whey protein hydrolysate, casein, casein hydrolysate, lactose, and mixtures thereof,
b. incubating said lactic acid producing bacteria in said aqueous medium, under aerobic or anaerobic conditions and at a temperature of approximately 20 °C to 50 °C, preferably for at least 2 h, and
c. inactivating and/or physically removing living cells of lactic acid producing bacteria from the aqueous medium.
   Optionally this process may be followed by one or more of the following steps:
d. ultrafiltrating the aqueous medium preferably through one or more filtration membranes having a cut-off threshold between 100 and 300 kDa, so as to obtain a concentrated retentate,
e. fractionating the concentrated retentate, preferably by dehydrating the concentrated retentate, dissolving the dehydrated retentate in a buffer, performing gel exclusion chromatography of the retentate solution, preferably on a column having an exclusion threshold of 600 kDa, and
f. recovering the desired fraction, preferably recovering the excluded fraction at the end of the chromatography, which fraction comprises or constitutes the milk-derived product.

The fermented product preferably comprises bacterial cell fragments like glycoproteins, glycolipids, peptidoglycan, lipoteichoic acid (LTA), flagellae, lipoproteins, DNA, and/or capsular polysaccharides. These fragments evoke an immunological response in the intestine of the newborn infant, thereby inducing tolerance and speed up fast bacterial colonisation of the intestine. It is of advantage to use the fermented milk-derived product comprising inactivated bacteria and/or cell fragments directly as a part of the final nutritional product, since this will result in a higher concentration of bacterial cell fragments. When commercial preparations of probiotics are used, these are usually washed and separated from the aqueous growth medium, comprising the bacterial cell fragments, thereby reducing or eliminating the presence of bacteria cell fragments. Furthermore, upon fermentation and/or other interactions of lactic acid producing bacteria with the milk substrate, additional bio-active compounds are formed, such as bioactive peptides and/or oligosaccharides, which also stimulate the immune system and/or stimulate the colonization of the intestinal microbiota. Therefore the fermented milk-derived product has an improved effect compared to non-fermented milk-derived product.

Preferably the nutritional composition comprises 5 to 100 wt.% of the fermented product based on dry weight, more preferably 10 to 100 wt.% , more preferably 20 to 90 wt.%, even more preferably 50 to 80 wt.%. Preferably, the composition comprises 1 to 100 wt% fermented product per 100 ml, more preferably 2 to 100 wt.%, more preferably 5 to 50 wt.%, even more preferably 10 to 20 wt.% per 100 ml.

### Lactic acid producing bacteria used for producing the fermented product

Lactic acid producing bacteria used for fermentation of the milk substrate are preferably provided as a mono- or mixed culture. Lactic acid producing bacteria consists of the genera *Bifidobacterium, Lactobacillus, Carnobacterium, Enterococcus, Lactococcus, Leuconostoc, Oenococcus, Pediococcus, Streptococcus, Tetragenococcus, Vagococcus* and *Weissella.* Preferably the lactic acid producing bacteria used for fermentation comprises bacteria of the genus *Lactobacillus* and/or *Bifidobacterium* and/or *Streptococcus.*

Bifidobacteria are Gram-positive, anaerobic, rod-shaped bacteria. The present *Bifidobacterium* species preferably have at least 95 % identity of the 16 S rRNA sequence when compared to the type strain of the respective *Bifidobacterium* species, more preferably at least 97% identity as defined in handbooks on this subject for instance Sambrook, J., Fritsch, E.F., and Maniatis, T. (1989), Molecular Cloning, A Laboratory Manual, 2nd ed., Cold Spring Harbor (N.Y.) Laboratory Press. The Bifodobacteria preferably used are also described by Scardovi, V. Genus Bifidobacterium, p.1418 - p.1434. In: Bergey's manual of systematic Bacteriology. Vol. 2. Sneath, P.H.A., N.S. Mair, M.E. Sharpe and J.G. Holt (ed.). Baltimore: Williams & Wilkins. 1986. 635 p. Preferably the lactic acid producing bacteria used for fermentation comprises or is at least one *Bifidobacterium* selected from the group consisting of *B. breve, B. infantis, B. bifidum, B. catenulatum, B. adolescentis, B. thermophilum, B. gallicum, B. animalis* or *lactis, B. angulatum, B. pseudocatenulatum, B. thermacidophilum* and *B. longum* more preferably *B. breve, B. infantis, B. bifidum, B. catenulatum, B. longum,* more preferably *B. longum* and *B. breve,* even more preferably *B. breve,* most preferably B. breve I-2219 deposited at the CNCM in Paris, France. Preferably the lactic acid producing bacteria used for fermentation comprises at least one, more preferably at least two, even more preferably at least three, most preferably at least four different *Bifidobacterium* species. Preferably the lactic acid producing bacteria used for fermentation comprises at least *B. longum* and/or *B. breve.* The above-mentioned combinations commonly aim to increase the tolerance against a more diverse quantity of microorganisms in the intestine of the caesarean section delivered infant. This beneficially affects the infant, proving numerous health benefits.

Lactobacilli are Gram-positive, anaerobic, rod-shaped bacteria. The present *Lactobacillus* species preferably have at least 95 % identity of the 16 S rRNA sequence when compared to the type strain of the respective *Lactobacillus* species, more preferably at least 97% identity as defined in handbooks on this subject for instance Sambrook, J., Fritsch, E.F., and Maniatis, T. (1989), Molecular Cloning, A Laboratory Manual, 2nd ed., Cold Spring Harbor (N.Y.) Laboratory Press. Preferably the lactic acid producing bacteria used for fermentation comprises at least one, more preferably at least two *Lactobacillus* species selected from the group consisting of *L. casei, L. reuteri, L paracasei, L. rhamnosus, L. acidophilus, L. johnsonii, L. lactis, L. salivarius, L. crispatus, L. gasseri, L. zeae, L. fermentum* and *L. plantarum,* more preferably *L. casei, L. paracasei, L. rhamnosus, L. johnsonii, L. acidophilus, L. fermentum* and even more preferably *L. paracasei.* Even more preferably the lactic acid producing bacteria used for fermentation comprises *Bifidobacterium breve* and/or *Lactobacillus paracasei,* because the growth of these bacteria in impaired in the intestine of formula fed infants, even when non-digestible oligosaccharides are added to the infant formula, compared to the intestine of breast fed infants. The further increased biodiversity will have a stimulatory effect on health of the newborn delivered by caesarean section.

Preferably the lactic acid producing bacteria used for fermentation comprises at least one microorganism selected from the group consisting of *Carnobacterium, Enterococcus, Lactococcus, Leuconostoc, Oenococcus, Pediococcus, Streptococcus, Tetragenococcus, Vagococcus* and *Weissella,* more preferably comprises or is *Streptococcus thermophilus.* The further increased biodiversity will have a stimulatory effect on health of the newborn delivered by caesarean section.

The inoculation density is preferably between 1x10² to 5x10¹⁰, preferably between 1x10⁴ to 5x10⁹ cfu lactic acid producing bacteria/ml aqueous medium containing milk substrate, more preferably between 1x10⁷ to 1x10⁹ cfu lactic acid producing bacteria/ml aqueous medium containing milk substrate. The final bacteria density after fermentation is preferably between 1x10³ to 1x10¹⁰, more preferably between 1x10⁴ to 1x10⁹ cfu/ml aqueous medium containing milk substrate.

Preferably the present nutritional composition comprises inactivated lactic acid producing bacteria and/or bacterial fragments derived from lactic acid producing bacteria obtained from more than 1x10² cfu lactic acid producing per g based on dry weight of the final composition, more preferably 1x10⁴cfu, even more preferably 1x10⁵ cfu. Preferably the inactivated bacteria or bacterial fragments are obtained from less than 1x10¹¹ cfu lactic acid producing bacteria per g based on dry weight of the final composition, more preferably 1x10¹⁰ cfu, even more preferably 1x10⁹ cfu.

### Profess of fermentation

The milk substrate to be fermented is selected from the group consisting of milk, whey, whey protein, whey protein hydrolysate, casein, casein hydrolysate, and lactose, and mixtures thereof, most preferably skimmed milk. Milk can be whole milk, semi-skimmed milk and/or skimmed milk. Whey can be sweet whey, and/or acid whey. Preferably the whey is present in a concentration of 3 to 80 g dry weight per l aqueous medium containing milk substrate, more preferably 40 to 60 g per l. Preferably whey protein hydrolysate is present in 2 to 80 g dry weight per l aqueous medium containing milk substrate, more preferably 5 to 15 g/l. Preferably lactose is present in 5 to 50 g dry weight per l aqueous substrate, more preferably 1 to 30 g/l. Preferably the aqueous medium containing milk substrate comprises buffer salts in order to keep the pH within a desired range. Preferably sodium or potassium dihydrogen phosphate is used as buffer salt, preferably in 0.5 to 5 g/l, more preferably 1.5 to3 g per l. Preferably the aqueous medium containing milk substrate comprises cysteine in amount of 0.1 to 0.5 g per l aqueous substrate, more preferably 0.2 to 0.4 g/l. The presence of cysteine results in low redox potential of the substrate which is advantageous for activity of lactic acid producing bacteria, particularly bifidobacteria. Preferably the aqueous medium containing milk substrate comprises yeast extract in an amount of 0.5 to 5 g/l aqueous medium containing milk substrate, more preferably 1.5 to 3 g/l. Yeast extract is a rich source of enzyme co-factors and growth factors for lactic acid producing bacteria. The presence of yeast extract will enhance the fermentation by lactic acid producing bacteria.

Suitably the aqueous medium containing milk substrate is pasteurised before the fermentation step, in order to eliminate the presence of unwanted living bacteria. Suitably the product is pasteurised after fermentation, in order to inactivate enzymes. Suitably the enzyme inactivation takes place at 75 °C for 3 min. Suitably the aqueous medium containing milk substrate is homogenised before and/or the milk-derived product is homogenised after the fermentation. Homogenisation results in a more stable substrate and/or fermented product, especially in the presence of fat.

The fermentation is preferably performed at a temperature of approximately 20 °C to 50 °C, more preferably 30 °C to 45 °C, even more preferably approximately 37 °C to 42 °C. The optimum temperature for growth and/or activity for lactic acid producing bacteria, more particularly lactobacilli and/or bifidobacteria is between 37 °C and 42 °C.

The incubation is preferably performed at a pH of 4 to 8, more preferably 6 to 7.5. This pH does not induce protein precipitation and/or an adverse taste, while at the same time lactic acid producing bacteria such as lactobacilli and/or bifidobacteria are able to ferment the milk substrate.

The incubation time is preferably at least 2 h, preferably between 4 and 48 h, more preferably between 6 and 24 h, even more preferably between 6 and 12 h. A sufficient long time enables the fermentation and the concomitant production of immunogenic cell fragments such as glycoproteins, glycolipids, peptidoglycan, lipoteichoic acid (LTA), flagellae, lipoproteins, DNA and/or capsular polysaccharides to take place at a high extent, whereas the incubation time need not be unnecessarily long for economical reasons.

Optionally one or more of the following steps may follow the above process:
i) Ultrafiltrating the milk-derived product after fermentation through filtration membranes having a cut-off threshold between 100 and 300 kDa, so as to obtain a concentrated retentate. The membranes are preferably polyethersulfone membranes and filtration is preferably performed at a temperature below 60 °C.
ii) Washing the concentrated retentate with water.
iii) Dehydrating the concentrated retentate, preferably by lyophilisation.
iv) Dissolving the dehydrated retentate in a buffer, preferably a Tris buffer with pH 6-8.
v) Performing gel exclusion chromatography of the retentate solution, on a column having an exclusion threshold of 600 kDa, preferably a Dextran or agarose column such as Superdex ®200.
vi) Desalting the product with a membrane with a cut-off of 10 kDa. Recovering the excluded fraction at the end of the chromatography, which fraction comprises or constitutes the milk-derived product.

Additional ingredients for producing the nutritional composition which is desired to obtain may be added. Usually these are added after step vi) above. For an infant milk formula, ingredients such as skimmed milk, whey, lactose, vegetable fat, mineral, vitamins etc as known in the art may be added.

Preferably, a milk substrate, preferably skimmed milk, is pasteurized, cooled and fermented with one or more *Lactobacillus* strains to a certain degree of acidity, upon which the fermented product is cooled and stored. Preferably a second milk-derived product is prepared in a similar way using one or more *Bifidobacterium* species for fermentation instead. Subsequently, the two fermented products are preferably mixed together and mixed with other components making up an infant formula, except the fat component. Preferably, the mixture is preheated, and subsequently fat is added in-line, homogenized, pasteurized and dried.

Preferably, a milk substrate, preferably lactose, is pasteurized, cooled and fermented with one or more *Streptococcus thermophilus* strains, upon which the fermented product is cooled and stored. Preferably a second milk-derived product is prepared in a similar way using skimmed milk and one or more *Bifidobacterium* species for fermentation instead. Subsequently, the two fermented products are preferably mixed together and mixed with other components making up an infant formula, pasteurized and dried.

A preferred method for preparing the fermented product of the present invention is disclosed in WO 01/01785, more particular in example 1 and 2. A preferred method for preparing the fermented product of the present invention is described in WO 2004/093899, more particularly in example 1.

### Methods of inactivation and/or physically removal of living cells

Living cells of lactic acid producing bacteria in the milk-derived product are after fermentation preferably essentially all eliminated, for example by inactivation and/or physical removal. The cells are preferably inactivated. Living bacterial cells are preferably inactivated by methods selected from the group consisting of heat treatment, UV treatment, sonication, treatment with oxygen, treatment with bactericidals such as ethanol, ultra high pressure application, high pressure homogenisation and/or use of a cell disruptor. Preferably the lactic acid producing bacteria are heat killed after fermentation of the milk-derived product. Preferable ways of heat killing are pasteurization, sterilization, ultra high temperature treatment, spray cooking and/or spray drying at temperatures bacteria do not survive. Cell fragments are preferably obtained by heat treatment, sonication, treatment with bactericidals such as ethanol, ultra high pressure application, high pressure homogenisation and/or use of a cell disruptor. Preferably intact cells of bacteria are removed from the fermented product by physical elimination such as filtration or centrifugation, for example centrifugation at 1 h at 3000 g, with the intact cells remaining in the pellet or retentate and the cell fragments remaining in the supernatant and/or filtrate, respectively. The inactivation and/or physical removal of living cells is such that the amount of living lactic acid producing bacteria is below detection limit as used by conventional plating techniques known in the art. This detection limit is less than 10³ cfu living cells of lactic acid producing bacteria based on g dry weight composition.

Inactivation of living cells has the advantage that, after production, the final nutritional composition can be pasteurised and/or sterilised, consequently reducing the chance of contamination with harmful micro-organisms, such as *E. sakazakii.* This is especially of importance for caesarean delivered infants since due to their delayed intestinal colonisation they are more prone to infections. So the present invention enables liquid, ready-to-use formula to be prepared and stored at room temperature. Furthermore, the dose of bioactive components received by each infant and/or toddler can be more easily controlled, since no further growth in a liquid product occurs, nor growth in the intestinal tract of the infant. The latter is a variable factor depending on the individual's intestinal environment, and thereby leads to variations in the extent of beneficial effects in individual infants.

Additional advantages are that the nutritional composition can be stored more easily and with reduced costs, since no special precautions have to be taken to maintain the viability of lactic acid producing bacteria at an acceptable level. This is especially the case in products with a water activity above 0.3. Also no post-acidification occurs in stored products with a high water activity and/or in infant formula in the period after reconstitution with water and before consumption. Adverse effect relating to coagulation of proteins and adverse taste are avoided in this way.

### Non-digestible oligosaccharides

The present composition preferably comprises at least two non-digestible oligosaccharides. The non-digestible oligosaccharide preferably stimulates the growth of the intestinal lactic acid producing bacteria, particularly bifidobacteria and/or the lactobacilli, more preferably bifidobacteria. The presence of non-digestible oligosaccharides stimulates the growth of lactic acid producing bacteria, such as lactobacilli and/or bifidobacteria, reduces the growth of non beneficial bacteria in the gastro-intestinal tract and/or directly advantageously stimulates the immune system. This results in a higher colonisation of the beneficial bacteria. Hence, the presence of both non-digestible oligosaccharides and the milk-derived product obtained by fermentation with lactic acid producing bacteria acts synergistically and advantageously results in both a faster and a higher colonization with lactic acid producing bacteria, such as lactobacilli and/or bifidobacteria.

The presence of at least two different non-digestible oligosaccharides results in a microbiota more diverse in respect of different lactic acid producing bacteria species as is the case in vaginally born infants. The presence of at least two different non-digestible oligosaccharides and the milk-derived product obtained by fermentation with lactic acid producing bacteria act synergistically and advantageously results in a faster, as well as a higher as well as a more diverse colonisation with lactic acid producing bacteria, especially with lactobacilli and/or bifidobacteria. The term "oligosaccharide" as used in the present invention refers to saccharides with a degree of polymerization (DP) of 2 to 250, preferably a DP 2 to 100, more preferably 2 to 60, even more preferably 2 to 10. If the oligosaccharide with a DP of 2 to 100 is included in the present composition, this includes compositions which contain oligosaccharides with a DP between 2 and 5, a DP between 50 and 70 and a DP of 7 to 60. The term "non-digestible oligosaccharide" as used in the present invention refers to oligosaccharides which are not digested in the intestine by the action of acids or digestive enzymes present in the human upper digestive tract (small intestine and stomach) but which are preferably fermented by the human intestinal microbiota. For example, sucrose, lactose, maltose and maltodextrins are considered digestible.

Preferably the present non-digestible oligosaccharide is soluble. The term "soluble" as used herein, when having reference to a polysaccharide, fibre or oligosaccharide, means that the substance is at least soluble according to the method described by L. Prosky et al., J. Assoc. Off. Anal. Chem. 71, 1017-1023 (1988).

Preferably, the present composition comprises non-digestible carbohydrate with a DP between 2 and 250, more preferably 2 to 60. The non-digestible carbohydrate are at least two selected from the group consisting of fructo-oligosaccharides, galacto-oligosaccharides, gluco-oligosaccharides, arabino-oligosaccharides, mannan-oligosaccharides, xylo-oligosaccharides, fuco-oligosaccharides, arabinogalacto-oligosaccharides, glucomanno-oligosaccharides, galactomanno-oligosaccharides sialic acid comprising oligosaccharides and uronic acid oligosaccharides. Preferably the present composition comprises fructo-oligosaccharides, galacto-oligosaccharides and/or galacturonic acid oligosaccharides, more preferably galacto-oligosaccharides, most preferably beta-galacto-oligosaccharides. The group of fructo-oligosaccharides includes inulin, the group of galacto-oligosaccharides includes transgalacto-oligosaccharides or beta-galacto-oligosaccharides, the group of gluco-oligosaccharides includes gentio-, nigero- and cyclodextrin-oligosaccharides and polydextrose, the group of arabinogalacto-oligosaccharides includes gum acacia, and the group of galactomanno-oligosaccharides includes partially hydrolysed guar gum.

The present composition preferably comprises at least two non-digestible oligosaccharides with different average degrees of polymerization (DP). Preferably the weight ratios:
a. (non-digestible oligosaccharides with DP 2 to 5) : (non-digestible oligosaccharides with DP 6, 7, 8, and/or 9) > 1; and/or
b. (non-digestible oligosaccharides with DP 10 to 60) : (non-digestible oligosaccharides with DP 6, 7, 8, and/or 9) > 1
Preferably both weight ratios are above 2, even more preferably above 5.

For further improvement, the present non-digestible oligosaccharide preferably has a relatively high content of short chain oligosaccharides, as these strongly stimulate the growth of bifidobacteria. Hence, preferably at least 10 wt.% of the non-digestible oligosaccharides in the present composition has a DP of 2 to 5 (i.e. 2, 3, 4, and/or 5) and at least 5 wt.% has a DP of 10 to 60. Preferably at least 50 wt.%, more preferably at least 75 wt.% of the non-digestible neutral oligosaccharides has a DP of 2 to 9 (i.e. 2, 3, 4, 5, 6, 7, 8, and/or 9).

More preferably the composition comprises galacto-oligosaccharides. The galacto-oligosaccharides are preferably selected from the group consisting of beta-galacto-oligosaccharides, lacto-N-tetraose (LNT), lacto-N-neotetraose (neo-LNT), fucosyl-lactose, fucosylated LNT and fucosylated neo-LNT. In a particularly preferred embodiment the present composition comprises beta-galacto-oligosaccharides. Beta-galacto-oligosaccharides as used in the present invention refers to oligosaccharides composed of over 50%, preferably over 65% galactose units based on monomeric subunits, with a degree of polymerization (DP) of 2 to 20, in which at least 50%, more preferably at least 75%, even more preferably at least 90%, of the galactose units are linked together via a beta-glycosidic linkage, preferably a beta-1,4 glycosidic linkage. Beta-linkages are also predominant in human milk oligosaccharides. The average DP is preferably of 3 to 6. A glucose unit may be present at the reducing end of the chain of galactose units. Beta-galacto-oligosaccharides are sometimes also referred to as transgalacto-oligosaccharides (TOS). A suitable source of beta-galacto-oligosaccharides is Vivinal®GOS (commercially available from Borculo Domo Ingredients, Zwolle, Netherlands). Other suitable sources are Oligomate (Yakult), Cupoligo, (Nissin) and Bi2muno (Classado). Beta-galacto-oligosaccharides were found to stimulate the growth of lactic acid producing bacteria, preferably bifidobacteria.

Preferably the composition comprises fructo-oligosaccharides. Fructo-oligosaccharides as used in the present invention refers to carbohydrates composed of over 50%, preferably over 65 % fructose units based on monomeric subunits, in which at least 50%, more preferably at least 75%, even more preferably at least 90%, of the fructose units are linked together via a beta-glycosidic linkage, preferably a beta-2,1 glycosidic linkage. A glucose unit may be present at the reducing end of the chain of galactose units. Preferably the fructo-oligosaccharide has a DP or average DP of 2 to 250, more preferably 2 to 100, even more preferably 10 to 60. Fructo-oligosaccaride comprises levan, hydrolysed levan, inulin, hydrolysed inulin, and synthesised fructo-oligosaccharides. Preferably the composition comprises short chain fructo-oligosaccharides with an average degree of polymerization (DP) of 3 to 6, more preferably hydrolysed inulin or synthetic fructo-oligosaccharide. Preferably the composition comprises long chain fructo-oligosaccharides with an average DP above 20, such as RaftilinHP. Preferably the composition comprises both short chain and long chain fructo-oligosaccharides. Fructo-oligosaccharide suitable for use in the compositions is also readily commercially available, e.g. RaftilineHP (Orafti).

More preferably the composition comprises a combination of galacto-oligosaccharides and fructo-oligosaccharides, more preferably long chain fructo-oligosaccharides. Such a mixture stimulates the growth of a healthy intestinal microbiota, particularly bifidobacteria and reduces the occurrence of *E. coli* in infants delivered via caesarean section. The mixture synergistically stimulates lactic acid producing bacteria, in particular bifidobacteria.

The present composition preferably comprises uronic acid oligosaccharides, more preferably galacturonic acid oligosaccharides. The term uronic acid oligosaccharide as used in the present invention refers to an oligosaccharide wherein at least 50% of the monosaccharide units present in the oligosaccharide is uronic acid. The term galacturonic acid oligosaccharide as used in the present invention refers to an oligosaccharide wherein at least 50% of the monosaccharide units present in the oligosaccharide is galacturonic acid. The galacturonic acid oligosaccharides used in the invention are preferably prepared from degradation of pectin, pectate, and/or polygalacturonic acid. Preferably the degraded pectin is prepared by hydrolysis and/or beta-elimination of fruit and/or vegetable pectins, more preferably apple, citrus and/or sugar beet pectin, even more preferably apple, citrus and/or sugar beet pectin degraded by at least one lyase. In a preferred embodiment, at least one of the terminal galacturonic acid units of the galacturonic acid oligosaccharide has a double bond. The double bond effectively protects against attachment of pathogenic bacteria to intestinal epithelial cells. Preferably one of the terminal galacturonic acid units comprises a C₄-C₅ double bond. The galacturonic acid oligosaccharide can be derivatised. The galacturonic acid oligosaccharide may be methoxylated and/or amidated. Preferably the galacturonic acid oligosaccharides are characterised by a degree of methoxylation above 20%, preferably above 50% even more preferably above 70%. Uronic acid oligosaccharides advantageously reduce the adhesion of pathogenic micro-organisms to the intestinal epithelial cells, thereby reducing colonization of (nosocomial) pathogenic bacteria in the colon of the infant delivered by caesarean section. Furthermore, uronic acid oligosaccharides preferably stimulate the formation of a healthy intestinal microbiota and are fermented, resulting in a production of intestinal organic acids and a reduction of intestinal pH, which inhibit the growth of (nosocomial) pathogenic bacteria.

Thus, in one embodiment the composition for use according to the present invention preferably comprises at least beta-galacto-oligosaccharides. In one embodiment the composition for use according to the present invention preferably comprises at least short chain fructo-oligosaccharides and/or long chain fructo-oligosaccharides, preferably long chain fructo-oligosaccharides. In one embodiment the composition for use according to the present invention preferably comprises at least uronic acid oligosaccharides. In one embodiment the composition for use according to the present invention preferably comprises at least beta-galacto-oligosaccharides and at least short chain fructo-oligosaccharides or long chain fructo-oligosaccharides or both. In one embodiment the composition for use according to the present invention preferably comprises at least beta-galacto-oligosaccharides and at least uronic acid oligosaccharides. In one embodiment the composition for use according to the present invention preferably comprises at least short chain fructo-oligosaccharides and uronic acid oligosaccharides or long chain fructo-oligosaccharides and uronic acid oligosaccharides. In one embodiment the composition for use according to the present invention preferably comprises at least beta-galacto-oligosaccharides and short chain fructo-oligosaccharides and uronic acid oligosaccharides or at least beta-galacto-oligosaccharides and long chain fructo-oligosaccharides and uronic acid oligosaccharides. A mixture of at least two different non-digestible carbohydrates advantageously stimulates the beneficial bacteria of the intestinal microbiota to a greater extent than based on the single non-digestible carbohydrates. A mixture of at least two different non-digestible carbohydrates advantageously stimulates a wider diversity of beneficial bacteria of the intestinal microbiota single non-digestible carbohydrates. Preferably the weight ratio between the mixture of two different non-digestible carbohydrates, preferably beta-galacto-oligosaccharides and fructo-oligosaccharide, is between 20 and 0.05, more preferably between 20 and 1. Beta-galacto-oligosaccharides are more reminiscent to the human milk oligosaccharides. Preferably the present composition comprises beta-galacto-oligosaccharides with a DP of 2-10 and/or fructo-oligosaccharides with a DP of 2-60. This combination was found to synergistically increase bifidobacteria and lactobacilli. The presence of these three non-digestible oligosaccharides even further stimulates the bifidobacteria. The weight ratio transgalacto-oligosaccharide : fructo-oligosaccharide : pectin degradation product is preferably (20 to 2) : 1 : (1 to 20), more preferably (12 to 7) : 1 : (1 to 3).

Preferably, the composition comprises of 80 mg to 2 g non-digestible oligosaccharides per 100 ml, more preferably 150 mg to 1.50 g, even more preferably 300 mg to 1 g. Based on dry weight, the composition preferably comprises 0.25 wt.% to 20 wt.%, more preferably 0.5 wt.% to 10 wt.%, even more preferably 1.5 wt.% to 7.5 wt.%. A lower amount of non-digestible oligosaccharides will be less effective in stimulating the beneficial bacteria in the microbiota, whereas a too high amount will result in side-effects of bloating and abdominal discomfort.

### Formulae

The composition used in the present invention are preferably nutritional and/or pharmaceutical compositions and suitable for administration to infants. The present composition is preferably enterally administered, more preferably orally.

The present composition is preferably a nutritional formula, preferably an infant formula. The present composition can be advantageously applied as a complete nutrition for infants. The present composition preferably comprises a lipid component, protein component and carbohydrate component and is preferably administered in liquid form. The present invention includes dry food (e.g. powders) which is accompanied with instructions as to mix said dry food mixture with a suitable liquid (e.g. water).

The present invention advantageously provides to a composition wherein the lipid provides 5 to 50% of the total calories, the protein provides 5 to 50% of the total calories, and the digestible carbohydrate component provides 15 to 90% of the total calories. Preferably, in the present composition the lipid provides 35 to 50% of the total calories, the protein provides 7.5 to 12.5% of the total calories, and the digestible carbohydrate provides 40 to 55% of the total calories. For calculation of the % of total calories for the protein, the total of energy provided by proteins, peptides and amino acids needs to be taken into account.

The present composition preferably comprises at least one lipid selected from the group consisting of animal lipid (excluding human lipids) and vegetable lipids. Preferably the present composition comprises a combination of vegetable lipids and at least one oil selected from the group consisting of fish oil, animal oil, algae oil, fungal oil, and bacterial oil. The present composition excludes human milk.

The protein used in the nutritional preparation is preferably selected from the group consisting of non-human animal proteins (preferably milk proteins), vegetable proteins (preferably soy protein and/or rice protein), hydrolysates thereof, free amino acids and mixtures thereof. The present composition preferably contains casein, whey, hydrolysed casein and/or hydrolysed whey protein. Preferably the protein comprises intact proteins, more preferably intact bovine whey proteins and/or intact bovine casein proteins. As the present composition is suitably used to reduce the allergic reaction in an infant, the protein of is preferably selected from the group consisting of hydrolyzed milk protein. Preferably the present composition comprises hydrolyzed casein and/or hydrolyzed whey protein, vegetable protein and/or amino acids. The use of these proteins further reduced the allergic reactions of the infant. The use of these hydrolysed proteins advantageously improves the absorption of the dietary protein component by the immature intestine of the infant delivered by caesarean section.

The present composition preferably comprises digestible carbohydrates selected from the group consisting of sucrose, lactose, glucose, fructose, corn syrup solids, starch and maltodextrins, more preferably lactose.

The present composition preferably has a viscosity between 1 and 60 mPa.s, preferably between 1 and 20 mPa.s, more preferably between 1 and 10 mPa.s, most preferably between 1 and 6 mPa.s. The low viscosity ensures a proper administration of the liquid, e.g. a proper passage through the whole of a nipple. Also this viscosity closely resembles the viscosity of human milk. Furthermore, a low viscosity results in a normal gastric emptying and a better energy intake, which is essential for infants which need the energy for optimal growth and development. The present composition is preferably prepared by admixing a powdered composition comprising with water. Normally infant formula is prepared in such way. The present invention thus also relates to a packaged power composition wherein said package is provided with instruction to admix the powder with a suitable amount of liquid, thereby resulting in a liquid composition with a viscosity between 1 and 60 mPa.s. The viscosity of the liquid is determined using a Physica Rheometer MCR 300 (Physica Messtechnik GmbH, Ostfilden, Germany) at a shear rate of 95 s⁻¹ at 20 °C.

Stool irregularities (e.g. hard stools, insufficient stool volume, and diarrhoea) are an important problem in babies delivered via caesarean section. This may be caused by the high content of *E. coli* in the faeces. It was found that stool problems may be reduced by administering the present non-digestible oligosaccharides in liquid food with an osmolality between 50 and 500 mOsm/kg, more preferably between 100 and 400 mOsm/kg. The reduced stool irregularities enhance the colonization and development of a healthy intestinal microbiota.

In view of the above, it is also important that the liquid food does not have an excessive caloric density, however still provides sufficient calories to feed the subject. Hence, the liquid food preferably has a caloric density between 0.1 and 2.5 kcal/ml, even more preferably a caloric density of between 0.5 and 1.5 kcal/ml, most preferably between 0.6 and 0.8 kcal/ml.

### Application

The present invention provides in one embodiment an enteral nutrional composition according to the present invention for use in administration to caesarean delivered infants. Preferably, the present invention provides (i) the treatment and/or prevention of a disorder in infants delivered via caesarean section and/or (ii) the stimulation of health in infants delivered via caesarean section. The disorder is preferably selected from the group consisting of intestinal disorders caused by a microbiota low in bifidobacteria. Preferably the disorder is selected from the group of allergy, eczema, asthma, infection and diarrhoea.

In one aspect the present invention provides a nutritional composition according to the present invention for use in the treatment of a disorder selected from the group consisting of allergy, eczema, asthma, infection and diarrhoea.

Providing the new-born caesarean section delivered infant with the milk-derived product, which is a milk substrate that is fermented by lactic acid producing bacteria, comprising immunogenic factors (inactivated cells and/or bacteria fragments such as glycoproteins, glycolipids, peptidoglycan, lipoteichoic acid (LTA), flagellae, lipoproteins, DNA and/or capsular polysaccharides), induces tolerance for those bacteria, thereby increasing intestinal colonization for these bacteria, and/or may decrease the colonization of adverse bacteria. These immunogenic factors may also have a direct effect on stimulating the growth of bifidobacteria and/or decreasing the growth of adverse bacteria. Induction of tolerance against bacteria in the intestinal tract results in a faster colonisation by the desired bacteria, while at the other hand the absence of living cells in the product results in an increased safety and improved product technological properties.

The present invention preferably provides a method for the prevention and/or treatment of infections and/or infection disorders, particularly gastrointestinal infections, more preferably the treatment and/or prevention of infections caused by one or more micro-organisms selected from the group consisting of *Staphylococcus (*especially *S. aureus, S. epidermidis, S. haemolyticus), Streptococcus* (especially *Streptococcus group B), Clostridium* (especially *C*. *difficile), Bacillus* (especially *B. subtilis, Pseudomonas* (especially *P. aeruginosa), Enterobacter, Klebsiella, Acinetobacter, Proteus, Aeromonas,* and *Escherichia,* preferably *Escherichia coli* (*E. coli*), said method comprising administering a nutritional composition according to the present invention.

Preferably, the present composition is used in a method for treatment and/or prevention of intestinal infection, intestinal inflammation and/or diarrhoea in infants delivered by caesarean section. Preferably the present composition is used in a method for modulating the immune system in infants born via caesarean section. In a further aspect, the present invention therefore provides a method for treatment and/or prevention of systemic infections, urinary tract infections, otitis and/or respiratory infections in infants delivered by caesarean section, said method comprising administering a nutritional composition according to the present invention.

In a further aspect, the present invention provides a method for treatment and/or prevention of allergy (particularly food allergy, more particularly cow's milk allergy), atopic eczema (e.g. atopic dermatitis), asthma, allergic rhinitis, and allergic conjunctivitis in infants delivered by caesarean section, even more preferably allergy and/or asthma, said method comprising administering to the infant a composition comprising the present milk-derived fermented product. These health effects are obtained by effects on immune system, intestinal wall and/or intestinal microbiota.

In the context of the present invention, 'prevention' of a disease or cetain disorder also means 'treatment of a person at risk' of said disease or said certain disorder.

Administration of the present composition results in an improved intestinal microbiota and subsequently in the formation of organic acids as metabolic end products of microbial fermentation. An increased amount of organic acids results in an increased mucus production, improves gut maturation and/or and increased gut barrier. Hence, in a further aspect, the present invention provides a method for decreasing intestinal wall permeability in caesarean section delivered infants and/or for improving intestinal wall maturation in caesarean section delivered infants, said method comprising administering to the infant a composition comprising derived product fermented by lactic acid producing bacteria.

Preferably the present composition is used for tolerance induction in the caesarean section delivered infant's intestine against bacteria and/or for improving intestinal colonisation of the microbiota in caesarean delivered infants towards the microbiota found in vaginally delivered infants and/or for a fast colonisation of a microbiota rich in lactic acid producing bacteria in caesarean section delivered infants.

The present invention also provides a method for stimulating the development of a healthy intestinal microbiota in an infant comprising step A: admixing I) a nutritionally or pharmaceutically acceptable liquid; and II) a dry composition, wherein the dry composition II comprises the present fermented milk-derived product; and step B) administering the composition obtained in step A to an infant born via caesarean section.

The present composition is preferably administered to the infant delivered via caesarean section in the first year of life, preferably within 3 months after birth, more preferably within six weeks after birth, even more preferably within two weeks after birth, even more preferably within one week after birth, more preferably within 72 hours, most preferably within 48 hours after birth.

### EXAMPLES

### Example 1: Effect of non-digestible oligosaccharides on the microbiota in caesarean delivered infants.

Infants were administered infant formula supplemented with 0.72 g/100ml galacto-oligosaccharides (GOS) with an average DP between 2 and 7 and 0.08 g/100ml fructo-oligosaccharides with an average DP of above 20 (FOS, Raftilin HP®) (GFSF-group) or standard infant formula without non-digestible oligosaccharides (SF-group).

The bifidobacterial content in the faeces was determined. The percentage of the genus *Bifidobacterium* as a percentage of total bacteria in the first week was 4.3% in caesarean delivered infants (n=44) versus 19.8% in vaginally delivered infants (n=28). At 6 weeks the percentage bifidobacteria was 12.3% in the SF-group of caesarean delivered infants (n=21) and 17.2% in the GFSF-group of the caesarean infants (n=13). The faecal pH of the SF-group of caesarean delivered infants was 7.2 versus 6.5 of the GFSF-group of caesarean delivered infants. The percentage *E. coli* was 11.8% in the SF group of caesarean delivered infants and 0% in the GFSF-group of the caesarean delivered infants. (See Table 1)

These results indicate that administration of non-digestible neutral oligosaccharides to infants born via caesarean section (GFSF group) results in a more bifidobacterial flora and a reduced content of potentially pathogenic bacteria compared to infants born via caesarean section that do not receive non-digestible oligosaccharides (the SF group). Additionally the results indicate a reduced content of *E. coli* due to the administration of non-digestible oligosaccharides. The results are indicative for the advantageous use of non-digestible oligosaccharides, particularly galacto-oligosaccharides and fructo-oligosaccharides in the present composition to further improve the present therapy in infants born via caesarean section.

**Table 1: Percentage Bifidobacteria in vaginally and caesarean section delivered infants fed a formula with (GFSF) or without (SF) non-digestible oligosaccharides.**

| Infants | *Bifidobacteria* in first week (%) | *Bifidobacteria* after 6 weeks (%) | Faecal pH | *E. coli* (%) |
|---|---|---|---|---|
| Vaginal delivery | 19.8 | | | |
| C-section SF | 4.3 | 12.3 | 7.2 | 11.8 |
| C-section GFSF | 4.3 | 17.2 | 6.5 | 0 |

### Example 2: Preparation of a fermented milk-derived product comprising inactivated B. breve.

A fermented product with *B. breve* CNCM I-2219 culture was obtained by a two steps process. A starter culture in the form of concentrated pellets was added to pasteurized skimmed milk and incubated for 8 h at 37 °C under anaerobic conditions. During the process the number of *B. breve* cells increased to about 3x10⁹ cfu per ml and an acidification from about pH 6.7 to a pH of between 4.5 and 5.0 occurred, indicative for the process of fermentation taking place. Suitably cysteine in amount of 0.1 to 0.5 g per l and/or yeast extract in an amount of 0.5 to 5 g/l aqueous substrate, was present.
This fermented product was subsequently diluted 10 times into concentrated pasteurized skim milk (43 wt% dry matter) with a temperature of 37 °C. The initial pH of the milk was reduced to 6-6.1 by this dilution step. After incubating for 8 h at 37°C, in a tank with periodic stirring for 10 minutes every 2 hours, the pH stayed between 6-6.1 and the *B. breve* population was 10⁶ cfu/ml. The concentrate was pasteurized and subsequently spray-dried and called BbC50cf. The amount of living *B. breve* cells was below detection limit (< 10³ cfu/g).

### Example 3: Synergistic effect of the fermented milk derived product comprising inactivated B. breve and non-digestible oligosaccharides on Th1 response increase in a murine vaccination model.

### Methods:

The effect of diets comprising (a) BbC50cf prepared according to example 2; (b) a combination of galacto-oligosaccharides (Elixor) and fructo-oligosaccharides (Raftilin HP); and (c) a combination of (a) and (b), were tested in a mouse model wherein a response to an antigen is measured by a delayed-type hypersensitivity (DTH) response. This DTH response in the ears after local challenge with an antigen present in a vaccine is a measure of Th1 cell proliferation. During response to infection and/or vaccination Th1 cells proliferate in response to the challenge with the antigen. These Th1 cells infiltrate the ear when the ear is subsequently challenged with the antigen and cause swelling. Infiltration with the Th1 cells in the ear takes about 24 h and the swelling is therefore delayed. The more Th1 cells proliferated during initial vaccination and/or infection, the more DTH upon challenging with the antigen is observed.
The BbC50cf was lyophilized and used in the mouse diet in a final concentration of 3 wt.%. Non-digestible oligosaccharide mixture (GF) containing transgalacto-oligosaccharides (GOS) (source Vivinal-GOS (Borculo Domo Ingredients, Netherlands)) and fructo-oligosaccharide (FPS) (source RaftilineHP, Orafti, Tiense, Belgium) were used in a weight ratio GOS : FPS of 9 : 1. Diets containing 1 wt. % GF based on total weight of the mouse diet were tested. The effects of a combination of non-digestible oligosaccharides and *B. breve* fermented mixture (GF plus BbC50cf) was tested in a diet containing 1 wt. % GF and 3 wt.% BbC50cf based on total weight of the diet.

Female, 6 weeks old C57B1/6 mice (Harlan Nederland BV, Horst, the Netherlands) were group-housed under a regular 12 hours light/dark regime. Group size was 10 animals per group and 3 animals in the negative control groups. The animals were given semi-synthetic diets (Research Diet Services, Wijk bij Duurstede, the Netherlands). Control diets were made to the AIN93G specifications (Reeves et al (1993) J Nutrition 123 (11): 1923-31), oligosaccharide supplemented diets were based on these specifications.

Vaccinations were started after a period of 20 days of adaptation to the new housing and diets. At day 0, a blood sample was collected prior to vaccination. At day 1, the first vaccination was administered subcutaneously. After three weeks, a booster vaccination was given (day 22). Nine days after booster injection (day 31), basal ear thickness was measured with a Digimatic outside micrometer (Mitutoyo, Veenendaal, the Netherlands) and a delayed-type hypersensitivity (DTH) response was induced by injecting antigen solution i. c. (intracutaneous) in the mouse ear pinnae. 24 h thereafter (day 32), the DTH response was measured, a blood sample was taken and the mice were sacrificed. This is the ear thickness after 24 h subtracted with the ear thickness at t=0.

The vaccinations consisted of a 100 µl i. c. (intracutaneous) injection of a 1: 1 mix of antigen solution and Stimune adjuvant (Specol, Cedi-diagnostics BV, Lelystad, the Netherlands). The antigen solution was a 1: 100 dilution of Influvac 2002/2003 (Solvay Pharmaceuticals, Weesp, the Netherlands) in PBS. Influvac is a trivalent protein vaccine, containing 3x3011 g/ml haemagglutinin of three different influenza strains. For the DTH responses, mice were i. c. injected with 25 µl dialysed Influvac in both ears as a DTH challenge.

### Results:

The diets containing dosages of 1 wt.% GF or 3 wt.% BbC50cf both induced a small non-statistically significant increase in the DTH response. The combination of 1 wt. % GF and 3 wt. % BbC50cf induced a statistically significant increase of 89 % in the DTH response (see Table 2). As the effect is significantly higher than the DTH responses from diets containing the oligosaccharides alone, and also much higher than based on the additive effect of GF and BbC50cf, which can be calculated to be 49% increase of DTH, these results are indicative for the synergistic effect provided by the administration of non-digestible oligosaccharides and BbC50cf on Th1 response increase. The observed effect is indicative for the advantageous use of a combination of at least two different non-digestible carbohydrates and a product obtained by fermenting a milk-derived product comprising inactivated lactic acid producing bacteria in the present uses.

**Table 2: DTH response**

| Group: | Mean DTH µm (S.E.) | Δ DTH µm | Relative DTH |
|---|---|---|---|
| Sham | -1.7 (4.6) | 0 | 0 |
| Placebo | 67.6 (14.9) | 69.3 | 1.00 |
| GF | 73.55 (8.6) | 75.25 | 1.09 |
| BbC50cf | 95.55 (5.5) | 97.25 | 1.40 |
| GF+BbC50cf | 129.4* (17.1) | 131.1 | 1.89 |
| GF+BbC50cf theoretically | 101.50 | 103.2 | 1.49 |

| | | | |
|---|---|---|---|
| * significantly different from control (P < 0.01). | | | |

### Example 4: Synergistic effect of the fermented milk derived product comprising inactivated B. breve and non-digestible oligosaccharides on decrease of Th2 response in a murine allergy model.

### Methods

The effect of diets as in example 3, were tested in a mouse model wherein a response to an allergen is measured by an immediate type hypersensitivity (ITH) response. This ITH response in the ears after challenge with an allergen in the lungs is a measure of an increased Th2 response. During response to the allergen in the lungs, masts cell almost immediately degranulate systemically, including in the ears. These reactions all involve IgE, which in its turn requires a Th2 response during helper T cell development. Therefore an enhanced ITH is indicative of increased IgE and hence increased Th2 response.
Specific pathogen free male BALB/c mice were obtained from Charles River (Maastricht, the Netherlands). Food and water was provided *ad libitum* and the mice were used when 6-9 weeks of age. Ovalbumin (grade V) and acetyl-β-methylcholine chloride (methacholine) were purchased from Sigma Chemical Co. (St. Louis, MO, USA). Aluminium hydroxide (AlumImject) was purchased from Pierce (Rockford, IL, USA).
The same diets as in example 3 were tested for 14 days prior to OVA sensitisation until the end of the experiment. Mice were sensitised by two i.p. injections with 10 µg ovalbumin adsorbed onto 2.25 mg aluminium hydroxide in 100 µl saline or saline alone on days 0 and 7. Mice were challenged on days 35, 38, and 41 by inhalation of ovalbumin aerosols in a plexiglass exposure chamber for 20 minutes. The aerosols were generated by nebulising an ovalbumin solution (10 mg/ml) in saline using a Pari LC Star nebulizer (Pari respiratory Equipment, Richmond, VA, USA). ITH was measured after 1 h after challenge with the allergen ovalbumin.

### Results

The results on ITH are shown in table 3:

**Table 3: ITH response**

| Group: | Mean ITH µm (S.E.) | Δ ITH µm | Relative ITH |
|---|---|---|---|
| control | 104.3 (3.8) | 0 | 0 |
| Placebo | 184.7 (8.7) | 80.4 | 1 |
| GF | 159.8 (17.9)* | 55.5 | 0.69 |
| BbC50cf | 173.1 (20.3) | 68.8 | 0.86 |
| GF+BbC50cf | 136.6 (14.4)** | 32.3 | 0.40 |
| GF+BbC50cf theoretically | 151.7 | 47.4 | 0.59 |

| | | | |
|---|---|---|---|
| * significantly different from control (*P < 0.05 and ** P < 0.01). | | | |

The diets containing dosages of 1 wt.% GF or 3 wt.% BbC50cf both induced a small decrease in the ITH response. The combination of 1 wt. % GF plus 3 wt. % BbC50cf induced a statistically significant decrease of about 60 % in the ITH response (see Table 3). As the effect is significantly higher than the ITH responses from diets containing the oligosaccharides alone, and also much higher than based on the additive effect of GF and BbC50cf, which can be calculated to be about 40 % decrease of ITH, these results are indicative for the synergistic effect provided by the administration of non-digestible oligosaccharides and a milk-derived fermented product on lowering of the Th2 response.
The observed effect is indicative for the advantageous use of a combination of non-digestible oligosaccharides and a product obtained by fermenting a milk-derived substrate with bifidobacteria and subsequently inactivating and/or removing the bifidobacteria so that the count is below 1 10³ cfu/g in the present uses. The results of this experiment are an indication that the present invention can advantageously be used for support in prevention and/or treatment of in particular asthma, allergy, atopic dermatitis, allergic conjunctivitis, dust mite allergy, urticaria and allergic rhinitis.

### Example 5: Composition for babies born via caesarean section

An infant formula was prepared comprising per 100 g dry material: protein (80% casein and 20% whey) 13 g; vegetable fat 25.5 g; lactose 42.25 g; maltodextrin 16 g; minerals 3 g; vitamins 0.25 g.

Vegetable fat was added to cow's milk heated at 75°C. The mixture was homogenised in two stages, the first one at 200 kg.s/cm², the second at 50 kg.s/cm². Aqueous solutions of lactose and maltodextrin and vitamins and minerals were added. The composition was pasteurised at 115°C and concentrated by evaporation to 48% dry material. The concentrate was cooled to 37°C and fermented with 5% of a culture of *B.breve* 1-2219 containing 10⁹ bacteria/ml and incubated for 8 h at 37°C. Subsequently the concentrate was pasteurized again.

The concentrate was spray dried and adding 140 g per litre water provided a reconstituted infant milk formula. Non-digestible oligosaccharides were included to result in 0.72 g beta-galacto-oligosaccharides and 0.08 g inulin per 100 ml ready to drink formula. The package and/or supporting material accompanying the product indicates that the product can suitably be used to a) stimulate intestinal colonisation with beneficial bacteria, b) prevent and/or treat infection in infants delivered via caesarean section; and/or c) prevent and/or treat allergy in infants delivered via caesarean section.

### Example 6: Composition for babies born via caesarean section

An infant formula comprising protein 21 g/l, fat 24 g/l, carbohydrates 83 g/l, non-digestible oligosaccharides 8 g/l, minerals 5 g/l, vitamins 0.45 g/l was prepared.

Fat was added to UHT sterilised milk at 70°C and the mixture was homogenised in two stages, the first one at 200 kg/cm2, the second at 50 kg/cm2. At 37°C the mixture was inoculated with 1.5% of a culture of *B.breve* 1-2219 containing 1 to 5 x 10⁹ bacteria/ml and fermented for 8 h at 37°C. The mixture was then cooled to 5°C. The rest of the ingredients were dissolved in water and added to the resulting product. The composition comprised per 100 ml ready to drink formula 0.72 g beta-galacto-oligosaccharides and 0.08 g long chain and/or short chain inulin.

The resulting mixture was UHT sterilised at 140°C for 6 to 7 seconds and aseptically packed.

## Claims

1. A nutritional composition comprising
a) a milk-derived product, said milk-derived product being a milk substrate that is fermented by lactic acid producing bacteria and said milk substrate comprising at least one selected from the group consisting of milk, whey , whey protein, whey protein hydrolysate, casein, casein hydrolysate and/or lactose,
b) less than 10³ cfu lactic acid producing bacteria per g dry weight of the composition, and
c) at least two non-digestible oligosaccharide selected from the group consisting of fructo-oligosaccharides, galacto-oligosaccharides, gluco-oligosaccharides, arabino-oligosaccharides, mannan-oligosaccharides, xylo-oligosaccharides, fuco-oligosaccharides, arabinogalacto-oligosaccharides, glucomanno-oligosaccharides, galactomanno-oligosaccharides, sialic acid comprising oligosaccharides and uronic acid oligosaccharides.

2. Composition according to any of the preceding claims wherein said lactic acid producing bacteria comprises *Bifidobacterium breve* and/or *Streptococcus thermophilus,* preferably *Bifidobacterium breve,* more preferably *B. breve* CNCM I-2219.

3. Composition according to any of the preceding claims wherein the composition comprises galacto-oligosaccharides and/or fructo-oligosaccharides.

4. Composition according to any one of the preceding claims wherein the composition comprises 0.5 to 10 wt.% of non-digestible oligosaccharide based on dry weight of the composition.

5. Composition according to any one of the preceding claims wherein the composition comprises protein, carbohydrate and fat, wherein the protein provides 5 to 25 % of the total calories, the fat provides 25 to 60 % of the total calories and the digestible carbohydrate provides 30 to 70 % of the total calories.

6. Composition according to any of the preceding claims wherein the composition has a viscosity of 1 to 6 mPa.s at a shear rate of 95 s⁻¹ at 20 °C.

7. Composition according to any of the proceeding claims wherein the milk-derived product is obtainable by a process comprising the steps of:
a) inoculating lactic acid producing bacteria in an aqueous medium comprising milk substrate in amount of between 1x10² to 5x10¹⁰ cfu lactic acid producing bacteria/ml, said aqueous medium having a pH of approximately between 4 and 8, and comprising at least one milk substrate selected from the group consisting of milk, whey, whey protein, whey protein hydrolysate, casein, casein hydrolysate, and/or lactose,
b) incubating said mixture under aerobic or anaerobic conditions at a temperature of approximately 20 °C to 50 °C, for at least 2 h, and
c) inactivating and/or physically removing living cells of lactic acid producing bacteria from the aqueous medium.

8. Composition according to any one of the preceding claims, wherein the composition is suitably to be enterally administered to a caesarean section delivered infant within one week after birth.

9. Composition according to any of the preceding claims for use for one selected from the group consisting of:
I) the treatment of a disorder selected from the group consisting of allergy, eczema, asthma, infection and diarrhoea in infants delivered via caesarean section, and
II) intestinal tolerance induction against lactic acid producing bacteria in caesarean section delivered infants, and/or for use in improving the colonisation of a microbiota rich in lactic acid producing bacteria in caesarean section delivered infants.

10. A nutritional composition comprising
a) a milk-derived product, said milk-derived product being a milk substrate that is fermented by lactic acid producing bacteria and said milk substrate comprising at least one selected from the group consisting of milk, whey, whey protein, whey protein hydrolysate, casein, casein hydrolysate and/or lactose, and
b) less than 10³ cfu lactic acid producing bacteria per g dry weight of the composition, for use for one selected from the group consisting of:
I) the treatment of a disorder selected from the group consisting of allergy, eczema, asthma, infection and diarrhoea in infants delivered via caesarean section,
II) intestinal tolerance induction against lactic acid producing bacteria in caesarean section delivered infants, and/or for use in improving the colonisation of a microbiota rich in lactic acid producing bacteria in caesarean section delivered infants.

11. Composition for use according to claim 10 wherein the milk-derived product is obtainable by a process comprising the steps of:
a) inoculating lactic acid producing bacteria in an aqueous medium comprising milk substrate in amount of between 1x10² to 5x10¹⁰ cfu lactic acid producing bacteria/ml, said aqueous medium having a pH of approximately between 4 and 8, and comprising at least one milk substrate selected from the group consisting of milk, whey, whey protein, whey protein hydrolysate, casein, casein hydrolysate, and/or lactose,
b) incubating said mixture under aerobic or anaerobic conditions at a temperature of approximately 20 °C to 50 °C, for at least 2 h, and
c) inactivating and/or physically removing living cells of lactic acid producing bacteria from the aqueous medium.

12. The composition for use according to any of claims 10-11, further comprising protein, carbohydrate and fat, wherein the protein provides 5 to 25 % of the total calories, the fat provides 25 to 60 % of the total calories and the digestible carbohydrate provides 30 to 70 % of the total calories.

13. The composition for use according to any of claims 10-12, further comprising at least one non-digestible oligosaccharide selected from the group consisting of fructo-oligosaccharides, galacto-oligosaccharides, gluco-oligosaccharides, arabino-oligosaccharides, mannan-oligosaccharides, xylo-oligosaccharides, fuco-oligosaccharides, arabinogalacto-oligosaccharides, glucomanno-oligosaccharides, galactomanno-oligosaccharides, sialic acid comprising oligosaccharides and uronic acid oligosaccharides.

14. The composition for use according to claim 13 wherein the non-digestible oligosaccharide is selected from the group consisting of galacto-oligosaccharides and fructo-oligosaccharides.

15. The composition for use according to claim 13 or 14, comprising 0.5 to 10 wt.% of non-digestible oligosaccharide based on dry weight of the composition.

## Patentansprüche

1. Ernährungszusammensetzung, welche umfasst:
a) ein von Milch abgeleitetes Produkt, wobei das von Milch abgeleitete Produkt ein Milchsubstrat ist, das durch Milchsäure produzierende Bakterien fermentiert ist, wobei das Milchsubstrat wenigstens eines ausgewählt aus der Gruppe bestehend aus Milch, Molke, Molkeprotein, Molkeproteinhydrolysat, Kasein, Kaseinhydrolysat und/oder Laktose umfasst,
b) weniger als 10³ cfu Milchsäure produzierende Bakterien pro g Trockengewicht der Zusammensetzung, und
c) wenigstens zwei nicht verdauliche Oligosaccharide, die ausgewählt sind aus der Gruppe bestehend aus Frukto-Oligosacchariden, Galakto-Oligosacchariden, Gluko-Oligosacchariden, Arabino-Oligosacchariden, Mannan-Oligosacchariden, Xylo-Oligosacchariden, Fuko-Oligosacchariden, Arabinogalakto-Oligosacchariden, Glucomanno-Oligosacchariden, Galaktomanno-Oligosacchariden, Sialinsäure umfassenden Oligosacchariden und Uronsäure-Oligosacchariden.

2. Zusammensetzung nach einem der vorangehenden Ansprüche, wobei die Milchsäure produzierenden Bakterien *Bifidobacterium breve* und/oder *Streptococcus thermophilus* umfassen, bevorzugt *Bifidobacterium breve,* noch bevorzugter *B. breve* CNCM 1-2219.

3. Zusammensetzung nach einem der vorangehenden Ansprüche, wobei die Zusammensetzung Galakto-Oligosaccharide und/oder Frukto-Oligosaccharide umfasst.

4. Zusammensetzung nach einem der vorangehenden Ansprüche, wobei die Zusammensetzung 0,5 bis 10 Gew.-% nicht verdauliches Oligosaccharid bezogen auf die Trockenmasse der Zusammensetzung umfasst.

5. Zusammensetzung nach einem der vorangehenden Ansprüche, wobei die Zusammensetzung Protein, Kohlenhydrat und Fett umfasst, wobei das Protein 5 bis 25 % der gesamten Kalorien bereitstellt, das Fett 25 bis 60 % der gesamten Kalorien bereitstellt und das verdaubare Kohlenhydrat 30 bis 70 % der gesamten Kalorien bereitstellt.

6. Zusammensetzung nach einem der vorangehenden Ansprüche, wobei die Zusammensetzung eine Viskosität von 1 bis 6 mPa.s bei einer Schergeschwindigkeit von 95 s⁻¹ bei 20 °C aufweist.

7. Zusammensetzung nach einem der vorangehenden Ansprüche, wobei das von Milch abgeleitete Produkt durch ein Verfahren erhaltbar ist, das die Schritte umfasst:
a) Impfen von Milchsäure produzierenden Bakterien in einem wässrigen Medium umfassend Milchsubstrat in einer Menge von zwischen 1x10² bis 5x10¹⁰ cfu Milchsäure produzierende Bakterien/ml, wobei das wässrige Medium einen pH-Wert von etwa zwischen 4 und 8 aufweist und wenigstens ein Milchsubstrat umfasst, das ausgewählt ist aus der Gruppe bestehend aus Milch, Molke, Molkeprotein, Molkeproteinhydrolysat, Kasein, Kaseinhydrolysat und/oder Laktose,
b) Inkubieren der Mischung unter aeroben oder anaeroben Bedingungen bei einer Temperatur von etwa 20 °C bis 50 °C, für wenigstens 2 Stunden, und
c) Inaktivieren und/oder physisches Entfernen von lebenden Zellen der Milchsäure produzierenden Bakterien aus dem wässrigen Medium.

8. Zusammensetzung nach einem der vorangehenden Ansprüche, wobei die Zusammensetzung passend ist für enteralen verabreichung an einen durch Kaiserschnitt entbundenen Säugling innerhalb einer Woche nach Geburt.

9. Zusammensetzung nach einem der vorangehenden Ansprüche zur Verwendung in
I) der Behandlung einer Störung ausgewählt aus der Gruppe bestehend aus Allergie, Ekzem, Asthma, Infektionen und Diarrhoe bei durch Kaiser-schnitt entbundenen Säuglingen
II) Darmtoleranzinduktion gegen Milchsäure produzierende Bakterien bei durch Kaiserschnitt entbundenen Säuglingen und/oder zur Verwendung bei der Verbesserung der Besiedelung einer an Milchsäure produzierenden Bakterien reichen Mikrobiota bei durch Kaiserschnitt entbundenen Säuglingen.

10. Ernährungszusammensetzung, welche umfasst:
a) ein von Milch abgeleitetes Produkt, wobei das von Milch abgeleitete Produkt ein Milchsubstrat ist, das durch Milchsäure produzierende Bakterien fermentiert ist, und wobei das Milchsubstrat wenigstens eines ausgewählt aus der Gruppe bestehend aus Milch, Molke, Molkeprotein, Molkeproteinhydrolysat, Kasein, Kaseinhydrolysat und/oder Laktose umfasst, und
b) weniger als 10³ cfu Milchsäure produzierende Bakterien pro g Trockengewicht der Zusammensetzung,
zur Verwendung in
I) der Behandlung einer Störung ausgewählt aus der Gruppe bestehend aus Allergie, Ekzem, Asthma, Infektionen und Diarrhoe bei durch Kaiserschnitt entbundenen Säuglingen,
II) Darmtoleranzinduktion gegen Milchsäure produzierende Bakterien bei durch Kaiserschnitt entbundenen Säuglingen und/oder zur Verwendung bei der Verbesserung der Besiedelung einer an Milchsäure produzierenden Bakterien reichen Mikrobiota bei durch Kaiserschnitt entbundenen Säuglingen.

11. Zusammensetzung nach Anspruch 10, wobei das von Milch abgeleitete Produkt erhaltbar ist durch ein Verfahren, welches die Schritte umfasst:
a) Impfen von Milchsäure produzierenden Bakterien in einem wässrigen Medium umfassend Milchsubstrat in einer Menge von zwischen 1x10² bis 5x10¹⁰ cfu Milchsäure produzierende Bakterien/ml, wobei das wässrige Medium einen pH-Wert von etwa zwischen 4 und 8 aufweist und wenigstens ein Milchsubstrat umfasst, das ausgewählt ist aus der Gruppe bestehend aus Milch, Molke, Molkeprotein, Molkeproteinhydrolysat, Kasein, Kaseinhydrolysat und/oder Laktose,
b) Inkubieren der Mischung unter aeroben oder anaeroben Bedingungen bei einer Temperatur von etwa 20 °C bis 50 °C, für wenigstens 2 Stunden, und
c) Inaktivieren und/oder physisches Entfernen von lebenden Zellen der Milchsäure produzierenden Bakterien aus dem wässrigen Medium.

12. Zusammensetzung nach einem der Ansprüche 10-11, weiter umfassend Protein, Kohlenhydrat und Fett, wobei das Protein 5 bis 25 % der gesamten Kalorien liefert, das Fett 25 bis 60 % der gesamten Kalorien liefert und das verdaubare Kohlenhydrat 30 bis 70 % der gesamten Kalorien liefert.

13. Zusammensetzung nach einem der Ansprüche 10-12, weiter umfassend wenigstens ein nicht verdauliches Oligosaccharid ausgewählt aus der Gruppe bestehend aus Frukto-Oligosacchariden, Galakto-Oligosacchariden, Gluko-Oligosacchariden, Arabino-Oligosacchariden, Mannan-Oligosacchariden, Xylo-Oligosacchariden, Fuko-Oligosacchariden, Arabinogalakto-Oligosacchariden, Glucomanno-Oligosacchariden, Galaktomanno-Oligosacchariden, Sialinsäure umfassenden Oligosacchariden und Uronsäure-Oligosacchariden.

14. Zusammensetzung nach Anspruch 13, wobei das nicht verdauliche Oligosaccharid ausgewählt ist aus der Gruppe bestehend aus Galakto-Oligosacchariden und Frukto-Oligosacchariden.

15. Zusammensetzung nach Anspruch 13 oder 14, umfassend 0,5 bis 10 Gew.-% nicht verdauliches Oligosaccharid bezogen auf die Trockenmasse der Zusammensetzung.

## Revendications

1. Composition nutritionnelle comprenant
a) un produit dérivé du lait, ledit produit dérivé du lait étant un substrat de lait qui est fermenté par des bactéries produisant de l'acide lactique et ledit substrat de lait comprenant au moins l'un(e) choisi(e) dans le groupe consistant en lait, lactosérum, protéine de lactosérum, hydrolysat de protéine de lactosérum, caséine, hydrolysat de caséine et/ou lactose,
b) moins de 10³ cfu de bactéries produisant de l'acide lactique par g de poids sec de la composition, et
c) au moins deux oligosaccharides non digestibles choisis dans le groupe consistant en fructo-oligosaccharides, galacto-oligosaccharides, gluco-oligosaccharides, arabino-oligosaccharides, mannane-oligosaccharides, xylo-oligosaccharides, fuco-oligosaccharides, arabinogalacto-oligosaccharides, glucomanno-oligosaccharides, galactomanno-oligosaccharides, oligosaccharides comprenant de l'acide sialique et oligosaccharides d'acide uronique.

2. Composition selon l'une quelconque des revendications précédentes, dans laquelle les bactéries produisant de l'acide lactique comprennent *Bifidobacterium breve* et/ou *Streptococcus thermophilus,* de préférence *Bifidobacterium breve,* plus préférablement *B. breve* CNCM 1-2219.

3. Composition selon l'une quelconque des revendications précédentes, la composition comprenant des galacto-oligosaccharides et/ou des fructo-oligosaccharides.

4. Composition selon l'une quelconque des revendications précédentes, la composition comprenant 0,5 à 10% en poids d'oligosaccharides non digestibles exprimés sur la base du poids sec de la composition.

5. Composition selon l'une quelconque des revendications précédentes, la composition comprenant des protéines, des glucides et des graisses, dans laquelle les protéines fournissent 5 à 25 % des calories totales, les graisses fournissent 25 à 60 % des calories totales et les glucides digestibles fournissent 30 à 70 % des calories totales.

6. Composition selon l'une quelconque des revendications précédentes, la composition présentant une viscosité de 1 à 6 mPa.s à un taux de cisaillement de 95 s⁻¹ à 20 °C.

7. Composition selon l'une quelconque des revendications précédentes, dans laquelle le produit dérivé du lait peut être obtenu par un procédé comprenant les étapes suivantes :
a) l'inoculation de bactéries produisant de l'acide lactique dans un milieu aqueux comprenant du substrat de lait dans une quantité située entre 1 x 10² et 5 x 10¹⁰ cfu de bactéries produisant de l'acide lactique/ml, ledit milieu aqueux ayant un pH situé approximativement entre 4 et 8, et comprenant au moins un substrat de lait choisi dans le groupe consistant en lait, lactosérum, protéine de lactosérum, hydrolysat de protéine de lactosérum, caséine, hydrolysat de caséine et/ou lactose,
b) l'incubation dudit mélange dans des conditions aérobies ou anaérobies à une température située approximativement entre 20 °C et 50 °C, pendant au moins 2 h, et
c) l'inactivation et/ou l'élimination physique des cellules vivantes des bactéries produisant de l'acide lactique du milieu aqueux.

8. Composition selon l'une quelconque des revendications précédentes, la composition étant convient à administrer par voie entérale au nourrisson né par césarienne dans la semaine suivant la naissance.

9. Composition selon l'une quelconque des revendications précédentes pour son utilisation dans
I) le traitement d'un trouble choisi dans le groupe consistant en allergie, eczéma, asthme, infection et diarrhées chez des nourrissons nés par césarienne,
II) l'induction d'une tolérance intestinale contre les bactéries produisant de l'acide lactique chez des nourrissons nés par césarienne et/ou pour son utilisation dans l'amélioration de la colonisation d'un microbiote riche en bactéries produisant de l'acide lactique chez des nourrissons nés par césarienne.

10. Composition nutritionnelle comprenant
a) un produit dérivé du lait, ledit produit dérivé du lait étant un substrat de lait qui est fermenté par des bactéries produisant de l'acide lactique et ledit substrat de lait comprenant au moins l'un(e) choisi(e) dans le groupe consistant en lait, lactosérum, protéine de lactosérum, hydrolysat de protéine de lactosérum, caséine, hydrolysat de caséine et/ou lactose,
b) moins de 10³ cfu de bactéries produisant de l'acide lactique par g de poids sec de la composition,
pour son utilisation dans
I) le traitement d'un trouble choisi dans le groupe consistant en allergie, eczéma, asthme, infection et diarrhées chez des nourrissons nés par césarienne,
II) l'induction d'une tolérance intestinale contre les bactéries produisant de l'acide lactique chez des nourrissons nés par césarienne et/ou pour son utilisation dans l'amélioration de la colonisation d'un microbiote riche en bactéries produisant de l'acide lactique chez des nourrissons nés par césarienne.

11. Composition selon la revendication 10, dans laquelle le produit dérivé du lait peut être obtenu par un procédé comprenant les étapes suivantes :
a) l'inoculation de bactéries produisant de l'acide lactique dans un milieu aqueux comprenant du substrat de lait dans une quantité située entre 1 x 10² et 5 x 10¹⁰ cfu de bactéries produisant de l'acide lactique/ml, ledit milieu aqueux ayant un pH situé approximativement entre 4 et 8, et comprenant au moins un substrat de lait choisi dans le groupe consistant en lait, lactosérum, protéine de lactosérum, hydrolysat de protéine de lactosérum, caséine, hydrolysat de caséine et/ou lactose,
b) l'incubation dudit mélange dans des conditions aérobies ou anaérobies à une température située approximativement entre 20 °C et 50 °C, pendant au moins 2 h, et
c) l'inactivation et/ou l'élimination physique des cellules vivantes des bactéries produisant de l'acide lactique du milieu aqueux.

12. Composition selon l'une quelconque des revendications 10-11, comprenant en outre des protéines, des glucides et des graisses, dans laquelle les protéines fournissent 5 à 25 % des calories totales, les graisses fournissent 25 à 60 % des calories totales et les glucides digestibles fournissent 30 à 70 % des calories totales.

13. Composition selon l'une quelconque des revendications 10-12, comprenant en outre au moins un oligosaccharide non digestible choisi dans le groupe consistant en fructo-oligosaccharides, galacto-oligosaccharides, gluco-oligosaccharides, arabino-oligosaccharides, mannane-oligosaccharides, xylo-oligosaccharides, fuco-oligosaccharides, arabinogalacto-oligosaccharides, glucomanno-oligosaccharides, galactomanno-oligosaccharides, oligosaccharides comprenant de l'acide sialique et oligosaccharides d'acide uronique.

14. Composition selon la revendication 13, dans laquelle l'oligosaccharide non digestible est choisi dans le groupe consistant en galacto-oligosaccharides et fructo-oligosaccharides.

15. Composition selon la revendication 13 ou 14, comprenant 0,5 à 10% en poids d'oligosaccharides non digestibles exprimés sur la base du poids sec de la composition.
